# DEMANDE DE BREVET EUROPEEN

(11) **EP 2 193 783 A1**
(43) Date de publication de la demande: **09.06.2010**
(21) Numéro de dépôt: 09174677.6
(22) Date de dépôt: 30.10.2009
(51) Int. Cl.: A61K 8/49, A61Q 17/04

(54) **Utilisation d'un derive ester de 2-pyrrolidinone 4- carboxy comme solvant dans les compositions cosmetiques ; compositions cosmetiques les contenant**

(30) Priorité: 08.12.2008 FR 0858337
(71) Demandeur: L'Oréal, 75008 Paris (FR)
(72) Inventeur: Richard, Hervé, 93320 Gagny (FR); Muller, Benoit, 75010, Paris (FR)
(74) Mandataire: Miszputen, Laurent

(57) **Abrégé**

La présente invention porte sur l'utilisation d'au moins un dérivé ester de 2-pyrrolidinone 4-carboxy de formule (I) : 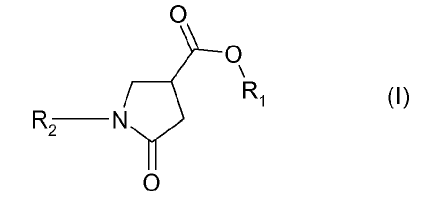 dans laquelle : .
R₁ désigne un radical alkyle en C₁-C₂₀, linéaire ou ramifié
R₂ désigne un radical alkyle en C₁-C₂₀, linéaire ou ramifié pouvant contenir un cycle en C₅-C₆, le radical phényle, le radical benzyle ou le radical phénéthyle, dans une composition comprenant dans un milieu cosmétiquement acceptable au moins une phase grasse liquide et au moins un actif lipophile, comme solvant dudit actif dans ladite phase grasse liquide et/ou comme agent améliorant la solubilité dudit actif dans ladite phase grasse.

La présente invention porte également sur une composition comprenant dans un milieu cosmétiquement acceptable au moins une phase grasse liquide caractérisée par le fait qu'elle contient au moins un dérivé ester de 2-pyrrolidinone 4-carboxy de formule (1) telle que défini ci-dessus et au moins un actif lipophile

## Description

La présente invention est relative à l'utilisation d'au moins un dérivé ester de 2-pyrrolidinone 4-carboxy de formule (I) dont on donnera la définition ci-après, dans une composition comprenant dans un milieu cosmétiquement acceptable au moins une phase grasse liquide et au moins un actif lipophile, comme solvant dudit actif dans ladite phase grasse liquide et/ou comme agent améliorant la solubilité dudit actif dans la phase grasse solide.

La présente invention concerne notamment une composition comprenant dans un milieu cosmétiquement acceptable au moins une phase grasse liquide caractérisée par le fait qu'elle contient au moins un dérivé ester de 2-pyrrolidinone 4-carboxy de formule (I) et au moins un actif lipophile.

De nombreux produits cosmétiques ou dermatologiques se présentent sous des formes galéniques diverses comprenant une phase grasse liquide telles que des dispersions, des lotions huileuses, des émulsions huile/eau, eau/huile ou multiple, des gels crèmes. Certains actifs cosmétiques ou dermatologiques particulièrement intéressants comme les filtres organiques lipophiles qui sont difficilement solubles dans la phase huileuse de ces formulations et qui ont tendance durant le stockage à former des cristaux ou à précipiter notamment dans les émulsions. De tels phénomènes sont indésirables d'un point de vue stabilité de la formulation et/ou vis-à-vis du confort du consommateur dans la mesure où ils peuvent déstabiliser la composition et/ou affecter l'apparence esthétique du produit et/ou entraîner des désagréments cosmétiques à l'application sur la peau et/ou les cheveux ou bien à limiter la concentration en actifs dans ces formules ce qui ne permet pas d'obtenir des produits suffisamment efficaces.

C'est le cas notamment des formulations de soin contenant des actifs lipophiles de faible solubilité tels que les actifs choisis parmi les dérivés d'aminophénol, les dérivés d'acide salicylique, les dérivés 2-amino 4-alkylaminopyrimidine 3-oxyde en particulier le 2-amino 4-dodécylaminopyrimidine 3-oxyde, la DHEA (déhydroépiandrostérone), ses dérivés et ses précurseurs chimiques tels que la 7-hydroxy ou 7 céto-DHEA, ou encore la 3β-acétoxy-7-céto-DHEA, le cholestérol et ses esters, les stérols végétaux tels que les phytostérols et les sitostérols et leurs esters, les acides triterpènes pentacycliques, les hydroxystilbènes, les flavonoïdes, les filtres UV organiques lipophiles dans les formulations antisolaires, le rétinol et ses dérivés, les carotenoïdes tel que le lycopène, et également les parfums, les huiles essentielles, les hormones, les vitamines en particulier la vitamine E, les céramides ou leurs mélanges.

En particulier, les compositions antisolaires se présentent souvent sous la forme d'une émulsion de type huile-dans-eau, ou eau-dans-huile, de gels ou de produits anhydres qui contiennent, à des concentrations diverses, un ou plusieurs filtres organiques et/ou inorganiques, insolubles et/ou solubles, lipophiles et/ou hydrophiles, capables d'absorber sélectivement le rayonnement UV nocif. Ces filtres et leurs quantités étant sélectionnés en fonction de l'indice de protection recherché. Selon leur caractère lipophile ou au contraire hydrophile, ces filtres peuvent se répartir, respectivement, soit dans la phase grasse, soit dans la phase aqueuse, de la composition finale.

Les filtres lipophiles sont couramment utilisés dans les formulations solaires. Cependant pour un certain nombre d'entre eux, leur pouvoir photoprotecteur en formulation est assez limité dans les supports cosmétiques habituels contenant des huiles telles que les (mono/poly)alcools gras oxyéthylénés ou oxypropylénés ("CETIOL HE" de chez Henkel ou "WITCONOL APM" de chez Witco) ou bien les esters gras tels que le benzoate d'alcools en C12-C15 ("FINSOLV TN" de chez Finetex), les triglycérides d'acides gras, par exemple le Miglyol® 812 commercialisé par la société DYNAMIT NOBEL, les dérivés d'acide aminés ("Eldew SL205" de chez Ajinomoto), à cause d'une solubilité de ces filtres dans ces huiles couramment utilisées en formulation pas complétement satisfaisante. Ceci a pour conséquence : soit l'apparition au cours du temps, de cristallisation dans les formules qui nuisent à la bonne qualité, stabilité et efficacité des produits solaires; soit à limiter la concentration en filtres dans les formules ce qui ne permet pas d'obtenir des produits suffisamment efficaces.

Il existe donc le besoin de trouver de nouveaux solvants permettant de dissoudre efficacement des actifs lipophiles et notamment les filtres lipophiles pour améliorer leur solubilité dans les huiles et dans les supports de formulations cosmétiques ou dermatologiques les contenant sans les inconvénients énumérés ci-dessus.

On sait que les dérivés de pyrrolidone sont connus comme agent de pénétration d'actifs comme l'oléocanthal dans la demande W02008/01240 ou comme les esters d'alkyl d'asprotadil dans le brevet US6,673,841.

Or, la Demanderesse vient maintenant de découvrir, de façon surprenante, une nouvelle famille de solvants efficaces constituée par des dérivés ester de 2-pyrrolidinone 4-carboxy de formule (I) dont on donnera la définition ci-après, permettant d'atteindre cet objectif. Ces composés peuvent être incorporés dans de nombreux produits cosmétiques.

Cette découverte est à la base de la présente invention.

La présente invention est relative à l'utilisation d'au moins un dérivé ester de 2-pyrrolidinone 4-carboxy de formule (I) dont on donnera la définition ci-après, dans une composition comprenant dans un milieu cosmétiquement acceptable au moins une phase grasse liquide et au moins un actif lipophile, comme solvant dudit actif dans ladite phase grasse liquide et/ou comme agent améliorant la solubilité dudit actif dans ladite phase grasse.

La présente invention concerne notamment une composition comprenant dans un milieu cosmétiquement acceptable au moins une phase grasse liquide caractérisée par le fait qu'elle contient au moins un dérivé ester de 2-pyrrolidinone 4-carboxy de formule (I) et au moins un actif lipophile.

La présente invention est relative également à l'utilisation d'au moins un dérivé de formule (I) dans une composition comprenant dans un milieu cosmétiquement acceptable au moins une phase grasse liquide et au moins un actif lipophile dans le but d'améliorer l'efficacité dudit actif et/ou les qualités cosmétiques et/ou la stabilité de ladite composition.

La présente invention est relative également à l'utilisation d'au moins un dérivé de formule (I) dans une composition comprenant dans un milieu cosmétiquement acceptable au moins une phase grasse liquide et au moins un filtre UV organique lipophile dans le but d'améliorer le facteur de protection solaire.

D'autres caractéristiques, aspects et avantages de l'invention apparaîtront à la lecture de la description détaillée qui va suivre.

Par "cosmétiquement acceptable", on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par "phase grasse liquide", au sens de la présente demande, on entend une phase grasse liquide à température ambiante (25°C) et pression atmosphérique (760mm de Hg), composée d'un ou plusieurs corps gras liquides à température ambiante, appelés aussi huiles, compatibles entre eux.

Par "actif lipophile" on entend tout actif organique cosmétique ou dermatologique susceptible d'être complètement dissous à l'état moléculaire dans une phase grasse liquide ou bien d'être solubilisé sous forme colloïdale (par exemple sous forme micellaire) dans une phase grasse liquide.

Par « actif lipophile de faible solubilité », on entend tout actif organique cosmétique ou dermatologique ayant une solubilité dans l'eau inférieure à 0,5 % en poids et une solubilité inférieure à 10 % en poids dans la plupart des solvants organiques comme l'huile de paraffine, les benzoates d'alcools gras et les triglycérides d'acides gras, par exemple le Miglyol® 812 commercialisé par la société DYNAMIT NOBEL. Cette solubilité, réalisée à 70°C est définie comme la quantité de produit en solution dans le solvant à l'équilibre avec un excès de solide en suspension après retour à la température ambiante. Elle peut facilement être évaluée au laboratoire.

Les dérivés esters de 2-pyrrolidinone 4-carboxy conformes à l'invention sont choisis par ceux répondant à la formule générale (I) suivante : 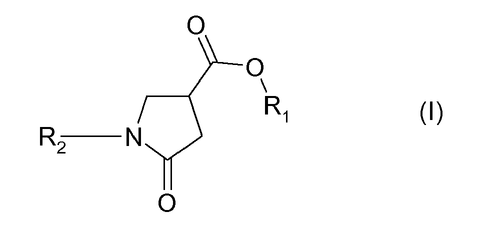 dans laquelle :
R₁ désigne un radical alkyle en C₁-C₂₀, linéaire ou ramifié
R₂ désigne un radical alkyle en C₁-C₂₀, linéaire ou ramifié pouvant contenir un cycle en C₅-C₆, le radical phényle, le radical benzyle ou le radical phénéthyle.

Dans la formule (I), parmi les groupes alkyles, on peut notamment citer les groupes méthyle, éthyle, n-propyle, isopropyle, n-butyle, iso-butyle, tert-butyle, n-octyle, éthyle-2 hexyle, dodécyle, héxadécyle, cyclohexyle ou méthyle cyclohexyle.

Parmi les composés de formule (I), on utilisera plus particulièrement les produits (a) à (oo) suivants :

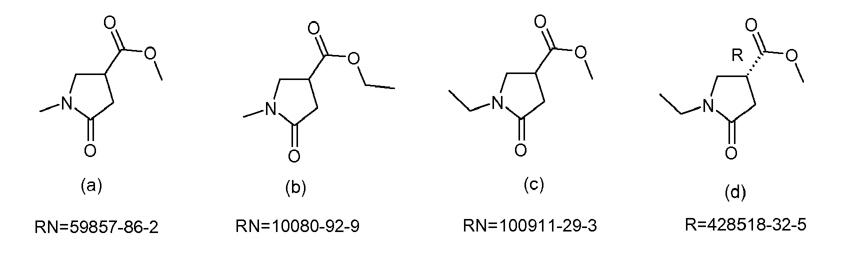

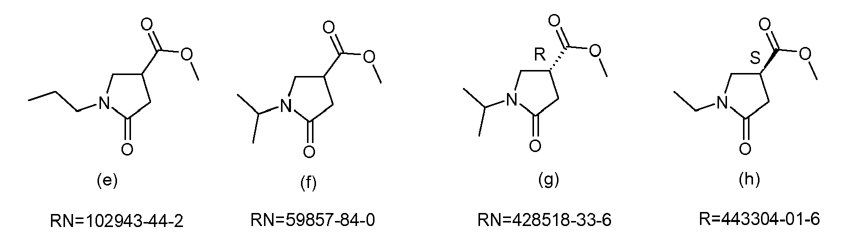

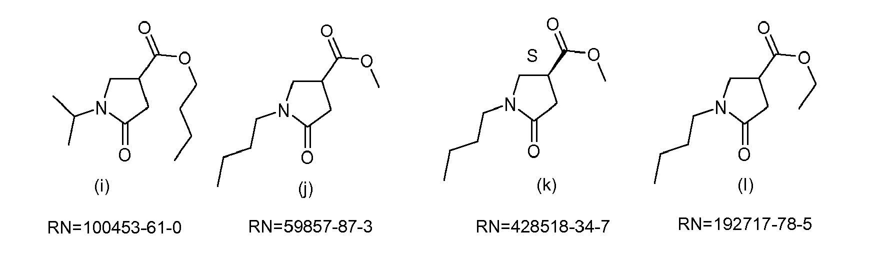

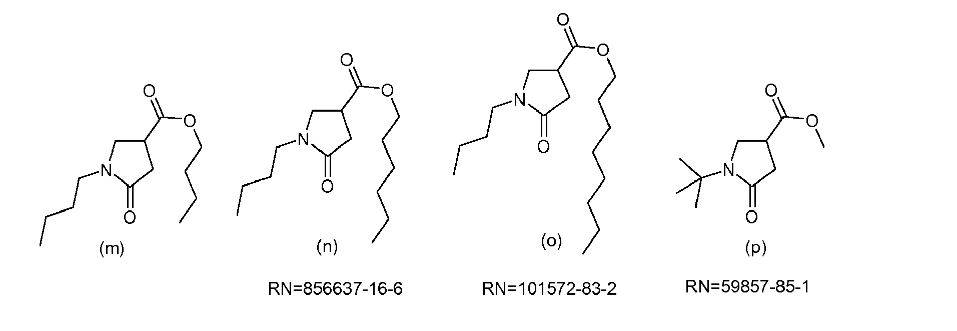

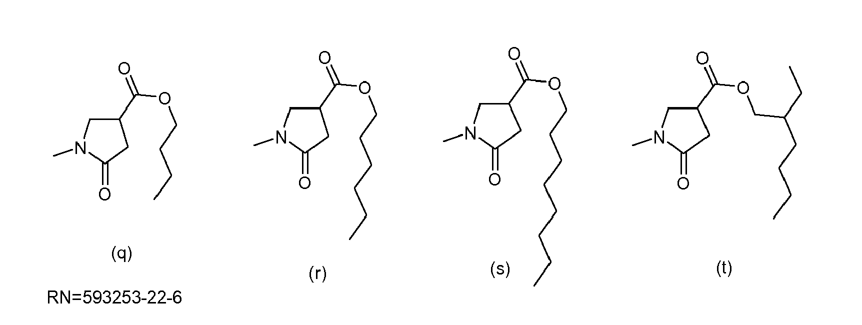

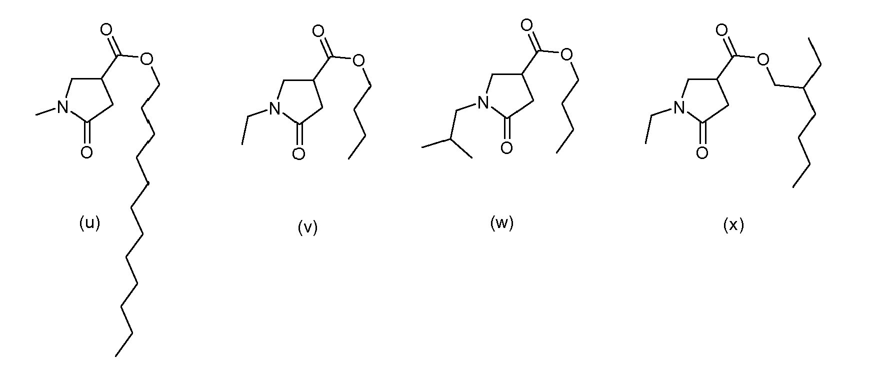

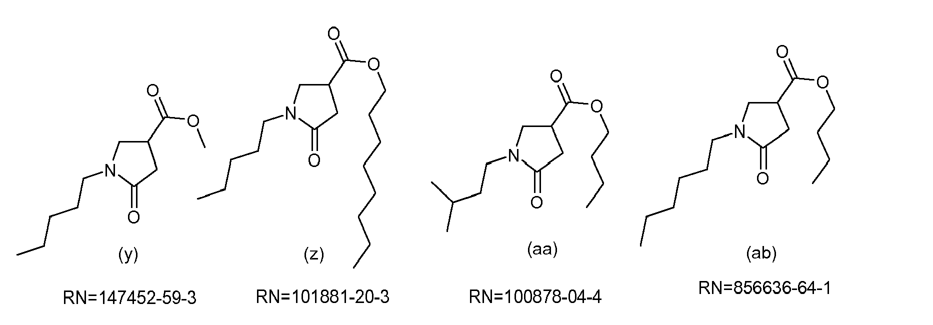

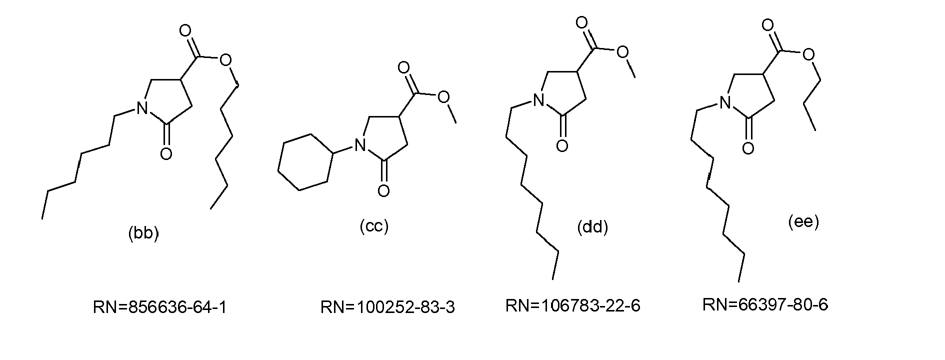

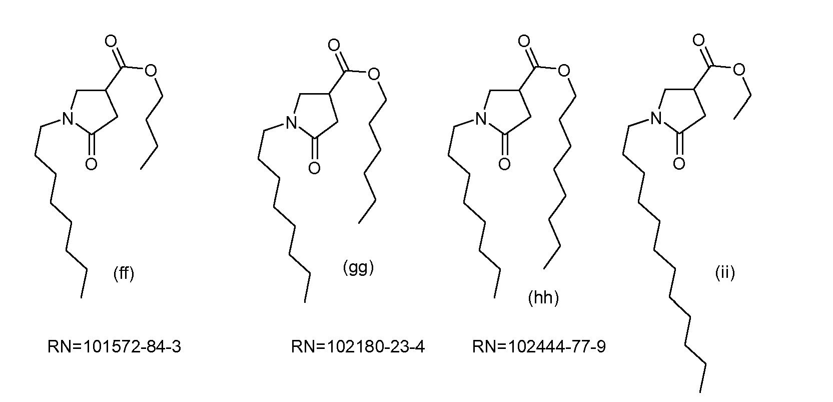

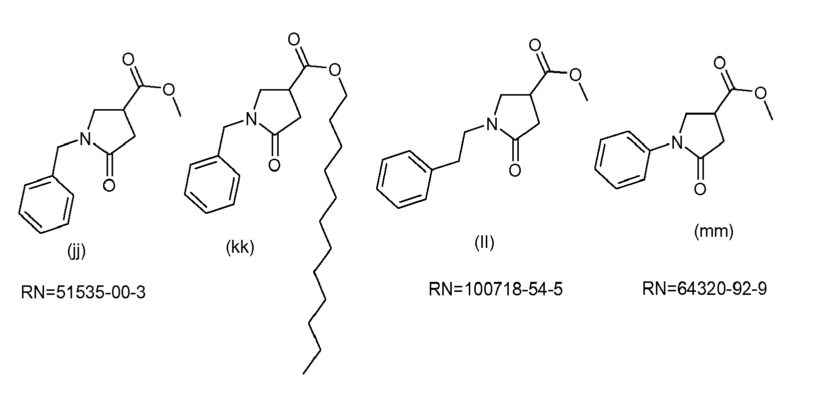

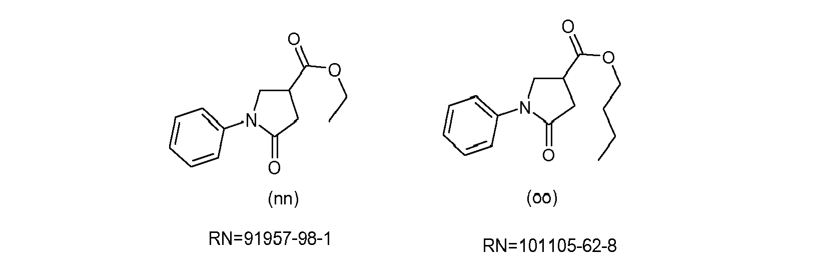

On utilisera plus particulièrement les dérivés des exemples (t), (u), (v), (x), (j), (I), (m), (w), (n), (ab), (ii) et (kk).

Les dérivés de formule (I) conformes à l'invention sont de préférence présents dans les compositions de l'invention à des teneurs allant de 1 à 30% en poids et plus préférentiellement de 3 à 20 % en poids par rapport au poids total de la composition.

Les dérivés de formule (I), dont les synthèses sont décrites dans les articles suivants : J. Org. Chem., 26, pages 1519-24 (1961) ; Tetrahedron Asymmetric, 12 (23), pages 3241-9 (2001) ; J. Industrial & Engineering Chem., 47, pages 1572-8 (1955) ; J. Am. Chem. Soc., 60, pages 402-6 (1938) ; et dans les brevets EP 0069512, US 2811496 (1955), US 2826588, US 3136620, FR 2290199 et FR 2696744 peuvent être facilement obtenus :
- soit par la condensation d'un diester de l'acide itaconique de formule (II) avec une amine primaire de formule (III), avec ou sans solvant à une température comprise entre 20°C et 150°C selon le schéma suivant : 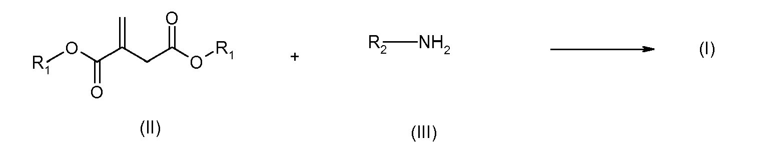
- soit en 2 étapes à partir de l'acide itaconique de formule (IV) par condensation avec l'amine primaire de formule (III) en présence d'un solvant ou pas pour donner l'acide intermédiaire de formule (V), suivi par une estérification de cet acide de formule (V) en présence d'un excès d'alcool de formule (VI) selon le schéma suivant : 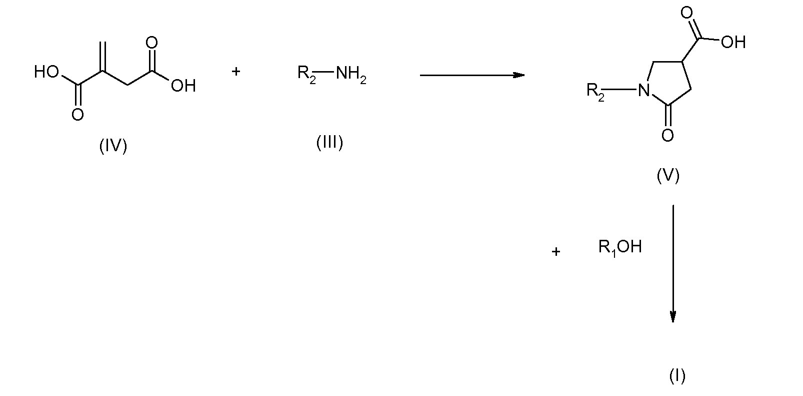
- soit les dérivés de formule (I) à chaîne ester R'₁ (radical alkyle en C₃-C₂₀, linéaire ou ramifié) peuvent également être obtenus par transestérification des dérivés à chaîne ester R₁ (radical méthyle ou éthyle) en présence d'alcool en C₃-C₂₀, linéaire ou ramifié et de catalyseur à l'étain ou au titane selon le schéma suivant :

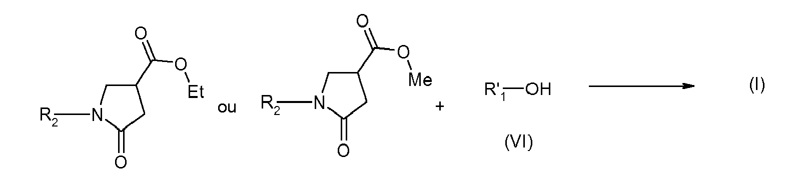

Selon une forme particulière de l'invention, le ou les dérivés de formule (I) conformes à l'invention sont présents dans une quantité suffisante permettant de solubiliser à lui-seul ou à eux-seuls (sans qu'il soit nécessaire d'utiliser un autre solvant) la quantité totale d'actif(s) lipophile (s) présent(s) dans la composition.

Selon autre une forme particulière de l'invention, le ou les dérivés de formule (I) conformes à l'invention constituent l'unique solvant du ou des actifs lipophile(s).

Dans ce cas la phase grasse liquide peut être constituée par le ou les dérivés de formule (I) et le ou les actifs lipophiles dissous dans ladite phase.

Parmi les actifs lipophiles utilisables conformément à l'invention, on utilisera des filtres organiques lipophiles. Ils peuvent être choisis parmi les dérivés de l'acide para-aminobenzoique, les dérivés salicyliques, les dérivés cinnamiques , les dérivés anthraniliques, les dérivés du dibenzoylméthane, les dérivés de β,β-diphénylacrylate, les dérivés du benzylidène camphre, les dérivés du phényl benzimidazole, les dérivés de benzotriazole, les dérivés d'imidazolines, les dérivés du benzalmalonate, les dérivés de 4,4-diarylbutadiène, les dérivés de benzoxazole, les dérivés de diphényl butadiène malonates ou malonitriles, les chalcones et leurs mélanges.

Parmi les filtres UVA lipophiles capables d'absorber les UV de 320 à 400 nm, on peut citer
Les dérivés du dibenzoylméthane :
   - le 4-isopropyl-dibenzoylméthane, vendu sous la dénomination de "EUSOLEX 8020" par la Société MERCK, et répondant à la formule suivante : 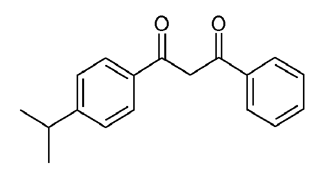
   - le 1-(4-methoxy-1-benzofuran-5-yl)-3-phenylpropane-1,3-dione, proposé à la vente par la société QUEST sous le nom de Pongamol de formule : 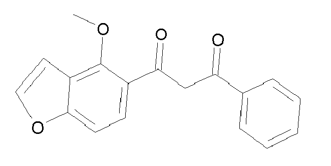
   - le 1-(4-tert-butylphenyl)-3-(2-hydroxyphenyl)propane-1,3-dione de formule : 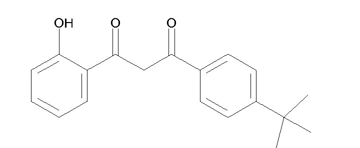
   - le Butyl Methoxydibenzoylmethane vendu notamment sous le nom commercial « PARSOL 1789 » par HOFFMANN LA ROCHE,
Les dérivés anthraniliques :
   Menthyl anthranilate vendu sous le nom commercial « NEO HELIOPAN MA » par HAARMANN et REIMER,
Les dérivés de 4,4-diarylbutadiène :
   1,1-dicarboxy (2,2'-diméthyl-propyl)-4,4-diphénylbutadiène
   Les préférentiels sont :
   Parmi les filtres UVB lipophiles capables d'absorber les UV de 280 à 320 nm , on peut citer
      Les para-aminobenzoates :
         Ethyl PABA
         Ethyl Dihydroxypropyl PABA
         Ethylhexyl Dimethyl PABA (ESCALOL 507 de ISP)
Les dérivés salicyliques :
   - Homosalate vendu sous le nom « Eusolex HMS » par Rona/EM Industries,
   - Ethylhexyl Salicylate vendu sous le nom « NEO HELIOPAN OS » par HAARMANN et REIMER,
   - Dipropyleneglycol Salicylate vendu sous le nom « DIPSAL » par SCHER, TEA Salicylate, vendu sous le nom « NEO HELIOPAN TS » par Haarmann et REIMER,
Les dérivés cinnamiques
   Ethylhexyl Methoxycinnamate vendu notamment sous le nom commercial « PARSOL MCX » par HOFFMANN LA ROCHE,
   Isopropyl Methoxy cinnamate,
   Isoamyl Methoxy cinnamate vendu sous le nom commercial « NEO HELIOPAN E 1000 » par HAARMANN et REIMER,
   Diisopropyl Methylcinnamate,
   Cinoxate,
   Glyceryl Ethylhexanoate Diméthoxycinnamate
   (2E)-3-(2,4,5-trimethoxyphenyl)prop-2-enoic acid de formule suivante : 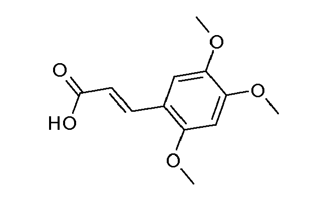
Les dérivés de β,β'-diphénylacrylate :
   Octocrylène, vendu notamment sous le nom commercial « UVINUL N539 » par BASF,
   Etocrylène, vendu notamment sous le nom commercial « UVINUL N35 » par BASF,
Les dérivés du benzylidène camphre :
   3-Benzylidène camphre fabriqué sous le nom « MEXORYL SD » par CHIMEX,
   Méthylbenzylidène camphre vendu sous le nom « EUSOLEX 6300 » par MERCK,
   Polyacrylamidométhyle Benzylidène Camphre fabriqué sous le nom « MEXORYL SW » par CHIMEX,
Les dérivés d'imidazolines :
   Ethylhexyl Dimethoxybenzylidene Dioxoimidazoline Propionate,
Les dérivés du benzalmalonate :
   Polyorganosiloxanes à fonction benzalmalonate tels que le Polysilicone-15 vendu sous la dénomination commerciale « PARSOL SLX » par HOFFMANN LA ROCHE
   Di-neopentyl 4'-méthoxybenzalmalonate,

Pami les filtres à spectre large lipophiles capables d'absorber les UVA et les UVB, on peut citer
Dérivés de benzotriazole :
   Drometrizole Trisiloxane vendu sous le nom « Silatrizole » par RHODIA CHIMIE Bumetrizole vendu sous le nom TINOGUARD AS par CIBA-GEIGY

### Dérivés de diphényl butadiènes :

Les dérivés de la famille des diphényl butadiènes malonates ou malonitriles sont les dérivés de formule générale (VII) suivante : 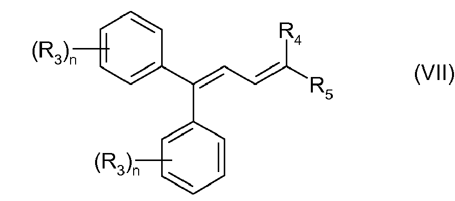 dans laquelle R₃ représente un groupement alkyle en C₁-C₂, un groupement alcoxy en C₁-C₂ et n est égal à 0, 1 ou 2 ;
R₄ et R₅, identiques ou différents représentent -COOR₆, -(C=O)NHR₆, -(C=O)R₆ , -CN dans lequel R₆ représente un groupement alkyle contenant de 1 à 12 atomes de carbone, linéaire ou ramifié et pouvant contenir des groupements silaniques, siloxaniques ou polysiloxaniques.

Parmi les dérivés de diphényl butadiène malonates ou malonitriles, on peut notamment citer, de manière non limitative :
- dimethyl 2-(3,3-diphenylprop-2-enylidene)malonate
- diisobutyl 2-(3,3-diphenylprop-2-enylidene)malonate
- bis(1,3-dimethylbutyl) 2-(3,3-diphenylprop-2-enylidene)malonate
- dineopentyl 2-(3,3-diphenylprop-2-enylidene)malonate
- methyl (2Z)-2-cyano-5,5-diphenylpenta-2,4-dienoate
- ethyl (trimethylsilyl)methyl (2Z)-2-(3,3-diphenylprop-2-enylidene)malonate
- (2E)-2-cyano-5,5-diphenyl-N-(3-{1,3,3,3-tetramethyl-1 - [(trimethylsilyl)oxy]disiloxanyl)propyl)penta-2,4-dienamide
- ethyl 2-methyl-3-{1,3,3,3-tetramethyl-1-[(trlmethylsllyl)oxy]disiloxanyl}propyl (2E)-2-(3,3-diphenylprop-2-enylidene)malonate
- ethyl (2Z)-5,5-diphenyl-2-{[(3-{1,3,3,3-tetramethyl-1-[(trimethylsilyl)oxy]disiloxanyl}propyl)amino]carbonyl}penta-2,4-dienoate

Parmi les dérivés de diphényl butadiènes mentionnés ci-dessus, on utilisera en particulier le dinéopentyl 2-(3,3-diphenylprop-2-enylidene)malonate répondant à la formule suivante :

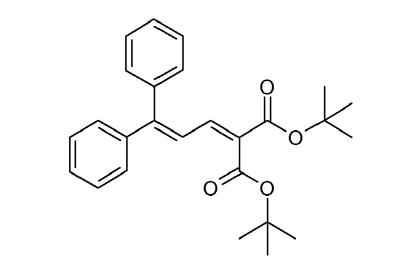

Il est connu d'utiliser ces dérivés de diphényl butadiènes dans des compositions solaires, le brevet EP 0916335 décrit les dérivés carbonés ainsi que ses modes d'obtention et les brevets EP 1535947 et EP 1535925 décrivent les dérivés siloxaniques et silaniques, respectivement.

### Les chalcones :

Les filtres lipophiles de la famille des chalcones répondent à la formule générale (VIII) suivante : 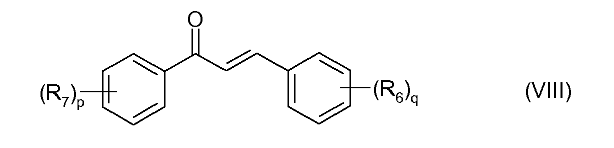 dans laquelle les radicaux R₆ et R₇ désignent indépendamment de chacun un atome d'hydrogène, le radical hydroxy, un groupement alkyle ou alcényle en C₁-C₁₂, linéaire ou ramifié, un groupement alcoxy en C₁-C₁₂, linéaire ou ramifié ou un groupe acyloxy en C₂-C₂₀, linéaire ou ramifié ;
petq

Parmi les dérivés de chalcones, on peut notamment citer, de manière non limitative :
- hydroxy-2' chalcone
- hydroxy-4' chalcone
- méthoxy-4' chalcone
- hydroxy-2' méthoxy-4 chalcone
- hydroxy-2' hexyloxy-4 chalcone
- hydroxy-2' méthyl-4 chalcone
- hydroxy-2' hexyloxy-3 chalcone
- hydroxy-2' hexyloxy-4' méthyl-4 chalcone
- hydroxy-2' hexanoyloxy-4' méthoxy-4 chalcone
- trihydroxy-2',4',4 diallyl-3,3' chalcone (connu sous le nom de Kazonol)
- trihydroxy-2',4',4 (methyl-3-but-2-ène)-5' chalcone (connu sous le nom de Broussochalcone B)
- pentahydroxy-2',3',4',6',4 chalcone (connu sous le nom de Carthamin)

Parmi les dérivés de chalcone mentionnés ci-dessus, on utilisera en particulier l'hydroxy-4' chalcone répondant à la formule (VIIIa) suivante : 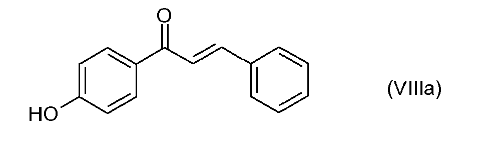 ou encore le pentahydroxy-2',3',4',6',4 chalcone (connu sous le nom de Carthamin) répondant à la formule (VIIIb) suivante :

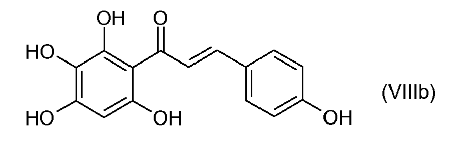

Il est connu d'utiliser ces composés chalcones dans des compositions solaires, notamment dans les brevets FR 2555167, FR 2602228 et FR 2608150.

Les filtres lipophiles sont généralement présents dans les compositions selon l'invention dans des proportions allant de 0,01 à 20% en poids par rapport au poids total de la composition, et de préférence allant de 0,1 à 10% en poids par rapport au poids total de la composition.

Parmi les filtres difficilement lipophiles, on peut citer par exemple les dérivés de dibenzoylméthane, les dérivés de benzotriazoles, les chalcones, les dérivés de la famille des diphényl butadiènes malonates ou malonitriles

Par « filtre lipophile de faible solubilité », on entend tout actif organique cosmétique ou dermatologique ayant une solubilité dans l'eau inférieure à 0,5 % en poids et une solubilité inférieure à 10 % en poids dans la plupart des solvants organiques comme l'huile de paraffine, les benzoates d'alcools gras et les triglycérides d'acides gras, par exemple le Miglyol® 812 commercialisé par la société DYNAMIT NOBEL. Cette solubilité, réalisée à 70 °C est définie comme la quantité de produit en solution dans le solvant à l'équilibre avec un excès de solide en suspension après retour à la température ambiante. Elle peut facilement être évaluée au laboratoire.

Les actifs lipophiles de faible solubilité conformes à l'invention peuvent être aussi choisis parmi les dérivés d'aminophénol, les dérivés d'acide salicylique, les dérivés de N,N'-di(arylmethylene)ethylenediaminetriacetates, les dérivés 2-amino 4-alkylaminopyrimidine 3-oxyde, les flavonoïdes, le rétinol et ses dérivés, les carotenoïdes tel que le lycopène, et également les parfums, les huiles essentielles, les hormones, les vitamines en particulier la vitamine E, les céramides ou leurs mélanges.

Les dérivés d'aminophénol utilisés sont plus particulièrement les dérivés de formule (IX) suivante : 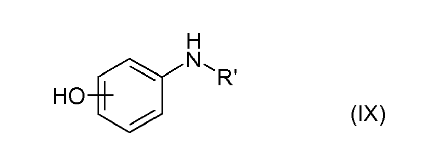 dans laquelle R' est un radical choisi dans le groupe formé par les radicaux (a), (b) et (c) suivants :
(a) -CO-NR¹R²
(b) -CO-O-R³
(c) -SO₂R³
où R¹ représente un atome d'hydrogène ou un radical alkyle en C₁ à C₆, linéaire ou ramifié, saturé ou insaturé, éventuellement hydroxylé,
R² représente un atome d'hydrogène ou un radical choisi parmi les radicaux alkyles, saturés ou insaturés, linéaires, cycliques ou ramifiés, en C₁₂ à C₃₀, éventuellement hydroxylé, et
R³ représente un radical choisi parmi les radicaux alkyles en C₁₂ à C₃₀, saturés ou insaturés, linéaires, cycliques ou ramifiés, y compris polycycliques condensés, et éventuellement hydroxylés.

Dans la formule (IX), parmi les radicaux R² ou R³ alkyles linéaires ou ramifiés ayant de 1 à 30 atomes de carbone, on peut citer avantageusement les radicaux méthyle, éthyle, propyle, isopropyle, butyle, tertiobutyle, hexyle, octyle, nonyle, 2-éthyl-hexyl, dodécyle, hexadécyle, béhényle et octadécyle, 2-butyl-octyle. De préférence, ces radicaux présentent de 1 à 12 atomes de carbone. De manière encore plus préférentielle, le radical alkyle comprend généralement de 1 à 6 atomes de carbone. On peut citer, comme radical alkyle inférieur, les radicaux méthyle, éthyle, propyle, isopropyle, tertiobutyle, hexyle.

Lorsqu'il est insaturé, on préfère un radical présentant une ou plusieurs insaturations éthyléniques, tels que plus particulièrement le radical allyle.

Lorsque le radical alkyle est cyclique, on peut notamment citer le radical cyclohexyle, cholestéryle ou terbutylcyclohexyle.

Lorsqu'il est hydroxylé, le radical comprend de préférence de 1 à 6 atomes de carbone et de 1 à 5 groupes hydroxyles. Parmi les radicaux monohydroxyalkyle, on préfère un radical contenant de préférence 1 ou 3 atomes de carbone, notamment les radicaux hydroxyméthyl, 2-hydroxyéthyl, 2 ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle et 2,3,4,5,6-pentahydroxyhexyle.

Les radicaux alkoxylés sont des radicaux alkyles, tels que notamment décrits ci-dessus, précédés d'un atome d'oxygène.

De préférence, les dérivés d'aminophénol utilisés dans la présente demande sont ceux pour lesquels l'une au moins et de préférence toutes les conditions ci-dessous sont respectées :
- la fonction -OH sur le radical phényl est en position ortho ou avantageusement en position para,
- R' est choisi parmi un radical de formules (a) ou (b).

Parmi les radicaux alkyle linéaire ou ramifié R¹, on peut citer les radicaux méthyle, éthyle, propyle, isopropyle, tertiobutyle, hexyle.

Le dérivé d'aminophénol utilisé de préférence dans ladite composition est un dérivé de para-aminophénol, de manière encore plus préférée, il s'agira du N-éthoxycarbonyl-4-para-aminophénol de formule (IXa) : 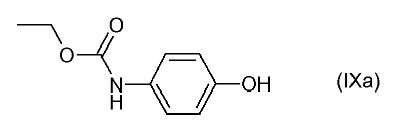 ou encore le N-cholestéryloxycarbonyl-4-para-aminophénol de formule (IXb) :

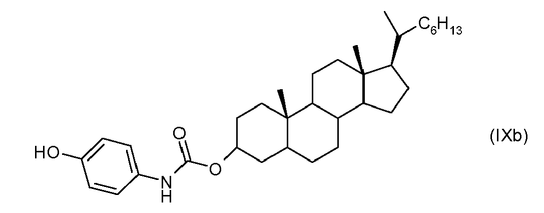

Ces dérivés d'aminophénol, ainsi que leur procédé de préparation, sont décrits dans les demandes de brevets WO 99/10318 et WO 99/32077.

Ces dérivés présentent une chaîne hydrocarbonée plus ou moins longue, de préférence alcoxycarbonyle, liée à l'atome d'azote. Ils présentent l'inconvénient d'être peu, voire pas du tout, solubles dans l'eau ni dans la phase grasse du type de celle utilisée dans la présente demande. Leur introduction dans des compositions cosmétiques nécessite, pour les composés à courte chaîne hydrocarbonée, une mise en solution hydroalcoolique, qui n'est pas toujours souhaitable lorsque la composition est destinée, par exemple, à être appliquée sur le contour de l'oeil.

De leur côté, les composés à longue chaîne hydrocarbonée sont insolubles dans les huiles, en raison de leur encombrement stérique, et ont tendance à recristalliser dans l'eau.

Les compositions selon la présente invention comprenant un tel dérivé d'aminophénol peuvent être utilisées comme agent dépigmentant ou blanchissant dans une composition cosmétique et/ou dermatologique.

Les dérivés d'acide salicylique sont les dérivés de formule (X) suivante : 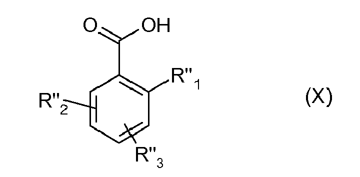 dans laquelle
R"₁ représente un radical hydroxyle ou un ester de formule -O-CO-R"₄
dans laquelle R"₄ est un radical aliphatique, saturé ou insaturé comprenant de 1 à 26 atomes de carbone, et de préférence de 1 à 18 atomes de carbone, une fonction amine ou thiol éventuellement substituée par un radical alkyle comprenant de 1 à 18 atomes de carbone, et de préférence de 1 à 12 atomes de carbone,
R"₂ et R"₃ indépendamment l'un de l'autre se trouvent en position 3, 4, 5 ou 6 sur le noyau benzénique et représentent indépendamment l'un de l'autre un atome d'hydrogène ou un radical : -(O)ₙ-(CO)ₘ-R"₅
dans lequel n et m, indépendamment l'un de l'autre, sont chacun un entier égal à 0 ou 1, à la condition que R"₂ et R"₃ ne soient pas simultanément des atomes d'hydrogène,
et R"₅ représente un hydrogène, un radical aliphatique saturé comprenant de 1 à 18 atomes de carbone, linéaire, ramifié ou cyclisé, un radical insaturé comprenant de 3 à 18 atomes de carbone, portant une à neuf doubles liaisons conjuguées ou non, les radicaux pouvant être substitués par au moins un substituant choisi parmi les atomes d'halogène (fluor, chlore, brome, iode), les radicaux trifluorométhyle, hydroxyle sous forme libre ou estérifiée par un acide comprenant de 1 à 6 atomes de carbone, ou carboxyle libre ou estérifié par un alcool inférieur comprenant de 1 à 6 atomes de carbone, un radical aromatique comprenant de 6 à 10 atomes de carbone.

De manière préférée, le dérivé d'acide salicylique est tel que R"₅ représente un radical aliphatique saturé comprenant de 3 à 15 atomes de carbone.

De préférence, le dérivé d'acide salicylique est tel que R"₁ représente un radical hydroxyle.

De préférence, le dérivé d'acide salicylique est tel que R"₅ est en position 5 du noyau benzénique et R"₂ représente un atome d'hydrogène.

Selon un mode de réalisation préféré de l'invention, il s'agit des dérivés d'acide n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, 5-tert-octylsalicylique, 3-tert-butyl-5-méthylsalicylique, 3-tert-butyl-6-méthylsalicylique, 3,5-diisopropylsalicylique, 5-butoxysalicylique, 5-octyloxysalicylique, propanoyl-5-salicylique, n-hexadecanoyl-5-salicylique, n-oléoyl-5-salicylique, benzoyl-5-salicylique, leurs sels monovalents et divalents et leurs mélanges.

On préfère tout particulièrement mettre en oeuvre le 2-hydroxy-5-octanoylbenzoic acid proposé à la vente sous la dénomination commerciale de "Mexoryl SAB" par la Société CHIMEX ; il répond à la formule (Xa) suivante :

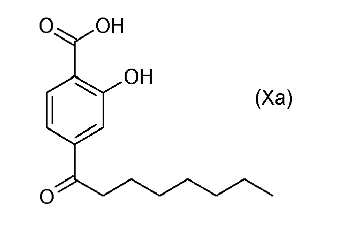

Il est connu d'utiliser des dérivés de l'acide salicylique dans des compositions topiques, par exemple, comme agent kératolytique pour traiter l'acné ou comme agent anti-vieillissement, les demandes de brevet FR-A-2 581 542 et EP-A-378 936 décrivent de tels dérivés.

Les dérivés d'acide salicylique sont d'un grand intérêt notamment pour prévenir ou réparer les principales manifestations du vieillissement cutané que sont les ridules et rides, la désorganisation du " grain " de la peau, la modification du teint de la peau et la perte de fermeté et de tonicité de la peau. Cependant, l'utilisation de ces dérivés pose un problème dans la mesure où, lorsqu'ils sont introduits tels quels dans les compositions topiques, ils ne se solubilisent pas et restent à l'état de cristaux, ce qui rend l'utilisation de la composition les contenant, inefficace pour le traitement de la peau.

Généralement, ces dérivés sont mis en solution dans les alcools inférieurs comme l'éthanol ou l'isopropanol ou des solvants tels que l'octyldodécanol, certains glycols, les alcools gras à chaîne courte (inférieurs à C₁₂). Cependant, ces alcools inférieurs présentent l'inconvénient de dessécher et d'irriter la peau ; on préfère donc éviter de les utiliser dans les produits de soin du corps et/ou du visage. En outre, ces solubilisants ne peuvent être introduits qu'en de petites quantités sous peine d'altérer les qualités cosmétiques (dessèchement de la peau) et la stabilité des compositions les contenant.

La concentration en dérivés d'acide salicylique de la composition selon la présente invention est de préférence de 0,001 à 15% en poids, plus préférentiellement de 0,1 à 5% en poids par rapport au poids total de la composition. La quantité d'esters d'acide aminé dépendra de la quantité de dérivés d'acide salicylique à solubiliser. Elle pourra être de 0,01 à 90% en poids, et de préférence entre 0,1 et 60% en poids par rapport au poids total de la composition.

La composition selon l'invention comprenant au moins un dérivé salicylique peut être utilisée comme composition cosmétique ou dermatologique, et notamment pour le soin, la protection, le nettoyage et/ou le maquillage des matières kératiniques des êtres humains (peau, lèvres, fibres kératiniques telles que cheveux et cils), et notamment pour lutter contre les signes du vieillissement cutané et/ou pour lisser la peau du visage et/ou du corps et/ou pour traiter les rides et les ridules de la peau et/ou pour stimuler le processus de renouvellement épidermique et/ou pour dépigmenter et blanchir la peau et/ou pour traiter l'acné et/ou pour traiter les désordres cutanés.

Ces dérivés ainsi que leur procédé de préparation, sont décrits dans le brevet WO 94/11338.

Les dérivés de la famille des 2-amino-4 alkylamino pyrimidine 3-oxydes sont les dérivés de formule générale (XI) suivante : 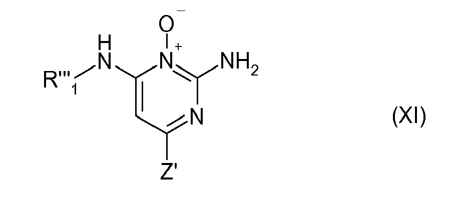 dans laquelle R"'₁ représente un groupement alkyle comprenant de 1 à 20 atomes de carbone, et Z' représente un atome d'hydrogène ou un radical - OR"'₂ dans lequel R"'₂ représente un groupement alkyle contenant de 1 à 12 atomes de carbone, ainsi que ses formes acylées ou ses sel d'addition avec les acides.

De préférence, R"'₁ est choisi dans le groupe formé par les radicaux hexyle, heptyle, octyle, nonyle, décyle, undécyle, dodécyle.

De préférence, R"'₂ est choisi dans le groupe formé par les radicaux éthyle, propyle, butyle, pentyle, hexyle.

De manière encore préférée, il s'agit du N-(2-amino-3-oxidopyrimidin-4-yl)-N'-dodecyl urea de formule (Xla) suivante :

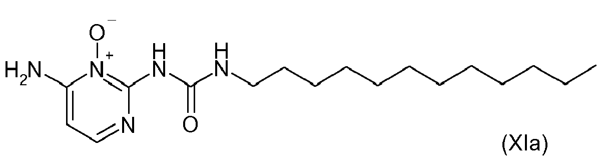

Les dérivés de la famille des 2-amino-4 alkylamino pyrimidine 3-oxydes peuvent notamment être utilisés dans ou pour la préparation d'une composition cosmétique ou dermatologique conforme à la présente invention pour prévenir et traiter les problèmes liés aux peaux sensibles et les perturbations cutanées telles que l'inconfort cutané, les tiraillements cutanés, les démangeaisons cutanées, le gonflement cutané, la rougeur cutanée et la sensation de chaleur cutanée.

Une autre famille de molécules qui entre dans la définition des molécules de faible solubilité dans l'eau est la DHEA, ses dérivés et ses précurseurs chimiques ou métaboliques

La DHEA ou déhydroépiandrostérone, également connue comme 3-beta-hydroxyandrost-5-en-17-one, ou déhydroisoandro-stérone, mais aussi trans-déhydro androstérone ou prastérone, a pour formule :

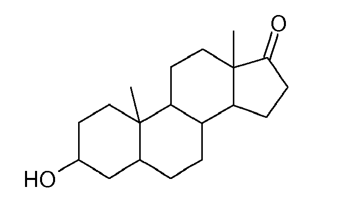

Par précurseurs de la DHEA concernés par l'invention, on entend ses précurseurs biologiques qui sont susceptibles de se transformer en DHEA au cours du métabolisme, ainsi que ses précurseurs chimiques qui peuvent se transformer en DHEA par réaction chimique exogène.

Des exemples de précurseurs biologiques sont la Δ5-prégnénolone, la 17α-hydroxy prégnénolone et le sulfate de 17α-hydroxy prégnénolone, sans que cette liste soit limitative.

Par précurseurs chimiques de la DHEA, on entend notamment les saponines et leurs dérivés tels que l'hécogénine (3 beta, 5 alpha, 23r)-3-hydroxyspirostan-12-one) et l'acétate d'hécogénine, la diosgénine (5-spirosten-3-beta-ol), le smilagénine et la sarsapogénine, ainsi que les extraits naturels en contenant, en particulier le fenugrec et les extraits de Dioscorées telles que la racine d'igname sauvage ou Wild Yam, sans que cette liste soit limitative.

Par dérivés de la DHEA, on entend aussi bien ses dérivés métaboliques que ses dérivés chimiques. Comme dérivés métaboliques, on peut citer notamment le Δ5-androstène-3,17-diol et notamment le 5-androstène 3β, 17β-diol, la Δ4-androstène-3,17-dione, la 7 hydroxy DHEA (7α-hydroxy DHEA ou 7β-hydroxy-DHEA) et la 7-céto-DHEA qui est elle-même un métabolite de la 7β-hydroxy DHEA, sans que cette liste soit limitative.

La 7α-hydroxy DHEA est, avec le 5-androstène 3 β., 17 β -diol, un métabolite majeur de la DHEA, obtenu par action de la 7α-hydroxylase sur la DHEA. La 7β-hydroxy DHEA est un métabolite mineur de la DHEA, obtenu par action de la 7β-hydroxylase sur la DHEA.

La 7-hydroxy DHEA utilisée de préférence dans les compositions selon la présente invention est la 7α-hydroxy DHEA. Un procédé de préparation de ce composé est décrit dans les demandes de brevet FR 2 771 105 et WO 94 08588.

En tant que dérivés chimiques de la DHEA, on peut encore citer les sels de DHEA et en particulier les sels hydrosolubles tel que le sulfate de DHEA ; les esters de DHEA tel que les esters d'acides hydrocarboxyliques et de DHEA en particulier ceux décrits dans le brevet US 5 736 537 ou encore le salicylate de DHEA, l'acétate de DHEA, le valérate (ou n-heptanoate) de DHEA et l'énanthate de DHEA.
On peut également citer les carbamates de DHEA, les esters de 2-hydroxy malonate de DHEA et les esters d'aminoacides de DHEA. Enfin, on peut citer la 3β-acétoxy-7-oxo-DHEA qui peut notamment être préparée comme décrit dans les brevets US-5 869 709 et US-6 111 118. Cette liste n'est évidemment pas limitative.

La concentration en composé à base de DHEA dans la composition selon la présente invention, peut aller avantageusement de 0,001 à 30% en poids, de préférence de 0,01 à 20%, et de manière encore plus préférée de 0,01 à 10% en poids par rapport au poids total de la composition. Ces composés seront sous forme solubilisée entre 20°C et 90°C.

Selon une forme particulière de l'invention, le ou les dérivés de formule (I) conformes à l'invention sont utilisés à titre d'unique solvant dudit ou desdits actifs lipophiles.

Les compositions conformes à l'invention peuvent comporter en plus d'autres filtres UV organiques UVA et/ou UVB hydrosolubles ou bien insolubles dans les solvants cosmétiques couramment utilisés.

Parmi les filtres hydrosolubles UVA capables d'absorber les UV de 320 à 400 nm , on peut citer
- Terephthalylidène Dicamphre Acide Sulfonic Acid fabriqué sous le nom "MEXORYL SX" par CHIMEX
- les dérivés bis-benzoazolyle tels que décrits dans les brevets EP 669 323, et US 2,463,264 et plus particulièrement le composé Disodium Phenyl Dibenzimidazole Tetra-sulfonate vendu sous le nom commercial commercial "NEO HELIOPAN AP" par Haarmann et REIMER,

Parmi les filtres hydrosolubles UVB capables d'absorber les UV de 280 à 320 nm, on peut citer les dérivés de p-aminobenzoïque (PABA) comme
PABA,
Glyceryl PABA, et
PEG-25 PABA vendu sous le nom "UVINUL P25" par BASF
Phenylbenzimidazole Sulfonic Acid vendu notamment sous le nom commercial "EUSOLEX 232" par MERCK,
Acide férulique
Acide salicylique
DEA methoxycinnamate
Benzylidène Camphre Acide Sulfonique fabriqué sous le nom "MEXORYL SL" par CHIMEX,
Camphre Benzalkonium Methosulfate fabriqué sous le nom "MEXORYL SO" par CHIMEX, et

Parmi les filtres hydrosolubles UVA et UVB, on peut citer
Benzophenone-4 vendu sous le nom commercial "UVINUL MS40" par BASF,
Benzophenone-5, et
Benzophenone-9.

Parmi les filtres insolubles , on peut citer
- Méthylène bis-Benzotriazolyl Tétramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
les filtres triazines symétriques décrits dans le brevet US 6,225,467, la demande WO 2004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphényl-4-yl-1,3,5-triazine) et la 2,4,6-tris(terphenyl)-1,3,5-triazine qui est repris dans les demandes de BEIERSDORF WO 06/035000, WO 06/034982, WO 06/034991, WO 06/035007, WO 2006/034992, WO 2006/034985.

Bien entendu, l'homme de l'art veillera à choisir le ou les éventuels filtres complémentaires et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.
Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les amides grasses (comme l'isopropyl lauroyl sarcosinate vendu sous la dénomination d' « Eldew SL-205 » par la société Ajinomoto), les acides ou les esters gras comme le benzoate d'alcools en C12-C15 vendu sous la dénomination commerciale « Finsolv TN » ou « Witconol TN » par la société WITCO, le Benzoate de 2-éthylphenyle comme le produit commercial vendu sous le nom X-TEND 226 ® par la société ISP, le palmitate d'octyle, le lanolate d'isopropyle, les triglycérides dont ceux des acides caprique/capryhque, le dicaprylyl carbonate vendu sous la dénomination « Cetiol CC » par la société Cognis), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la cire de carnauba, la cire d'abeille, l'huile de ricin hydrogénée, les cires de polyéthylène et les cires de polyméthylène comme celle vendue sous la dénomination Cirebelle 303 par la société SASOL.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les Carbopols (Carbomers) et les Pemulen (Copolymère acrylate/C10-C30-alkylacrylate); les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose , les polysaccharides et notamment les gommes telles que la gomme de Xanthane ; et leurs mélanges.

Comme épaississants lipophiles, on peut citer les polymères synthétiques tels que les poly C10-C30 alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Parmi les actifs, on peut citer :
- les vitamines (C, K, PP...) et leurs dérivés ou précurseurs, seuls ou en mélanges,
- les agents anti-pollution et/ou agent anti-radicalaire ;
- les agents dépigmentants et/ou des agents pro-pigmentants ;
- les agents anti-glycation ;
- les agents apaisants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants;
- les agents tenseurs,
- les agents matifiants,
- les agents kératolytiques,
- les agents desquamants ;
- les agents hydratants ;
- les agents anti-inflammatoires ;
- les agents agissant sur le métabolisme énergétique des cellules,
- les agents répulsifs contre les insectes
- les antagonistes de substances P ou de CRGP.
- les agents anti-chute et/ou repousse des cheveux
- les agents anti-rides.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsions peuvent contenir également d'autres types de stabilisants comme par exemple des charges, des polymères gélifiants ou épaississants.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange de cyclométhicone et de diméthicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostéarate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol.

Comme esters alkylés de polyol, on peut citer notamment les esters de polyéthylèneglycol comme le PEG-30 Dipolyhydroxystearate tel que le produit commercialisé sous le nom Arlacel P135 par la socité ICI .

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/Glyceryl Stearate commercialisé par exemple par la société ICI sous la dénomination Arlacel 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Parmi les autres stabilisants d'émulsion, on utilisera plus particulièrement les polymères d'acide isophtalique ou d'acide sulfoisophtalique, et en particulier les copolymères de phtalate / sulfoisophtalate / glycol par exemple le copolymère de Diéthylèneglycol / Phtalate / Isophtalate / 1,4-cyclohexane-diméthanol (nom INCI : Polyester-5) vendu sous les dénominations "Eastman AQ polymer" (AQ35S, AQ38S, AQ55S, AQ48 Ultra) par la société Eastman Chemical.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517.

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Des exemples concrets, mais nullement limitatifs, illustrant l'invention, vont maintenant être donnés.

### SOLUBILITES COMPAREES D'ACTIFS LIPOPHILES ENTRE DES SOLVANTS DE L'ART ANTERIEUR ET LES SOLVANTS DE L'INVENTION

### Actifs testés :

**Actif 1** : Ethyl 4-hydroxyphenylcarbamate 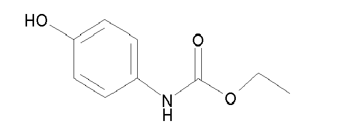
**Actif 2** : 2-hydroxy-5-octanoylbenzoic acid 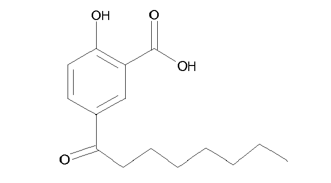
**Actif 3** : N-(2-amino-3-oxidopyrimidin-4-yl)-N'-dodecylurea

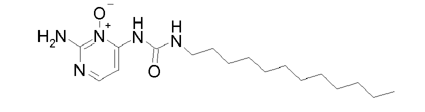

### Filtres testés

**Fil 1** : 1-(4-tert-butylphenyl)-3-(2-hydroxyphenyl)propane-1,3-dione 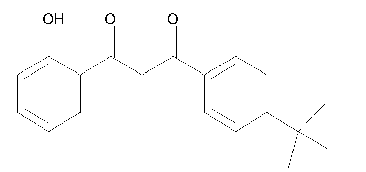
**Fil 2** : 1-(4-methoxy-1-benzofuran-5-yl)-3-phenylpropane-1,3-dione 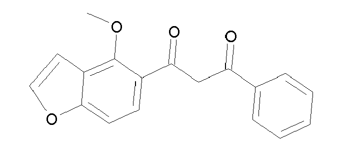
**Fil 3** : 1-(4-tert-butylphenyl)-3-(4-methoxyphenyl)propane-1,3-dione (avobenzone) (connu sous le nom de Parsol 1789 de DSM) : 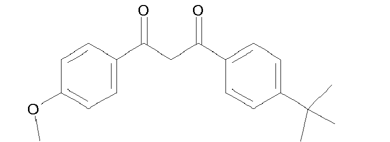
**Fil 4** : 1-(4-isopropylphenyl)-3-phenylpropane-1,3-dione : 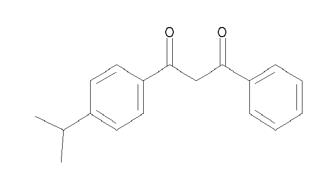
**Fil 5** : dineopentyl 2-(3,3-diphenylprop-2-enylidene)malonate : 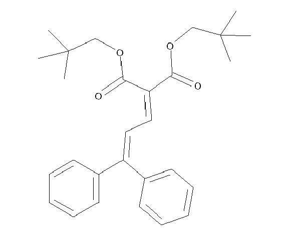
**Fil 6** : 2-(2H-1,2,3-benzotriazol-2-yl)-4-methyl-6-(2-methyl-3-{1,3,3,3-tetramethyl-1-[(trimethylsilyl)oxy]disiloxanyl}propyl)phenol : 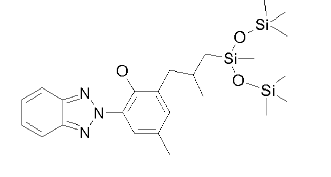
**Fil 7** : (2E)-3-(2,4,5-trimethoxyphenyl)prop-2-enoic acid : 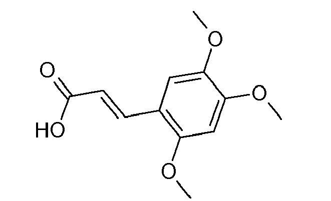
**Fil 8** : (2E)-1-(4-hydroxyphenyl)-3-phenylprop-2-en-1-one : 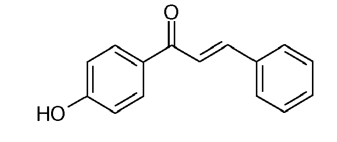
**Huile comparative 1** : Eldew SL-205 d' Ajinomoto : Isopropyl N-lauroyl sarcosinate de formule : 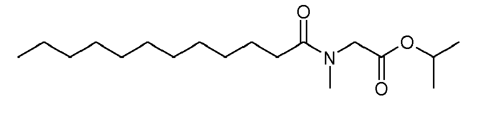
**Huile comparative 2 :** Finsolv TN : Alkylbenzoate en C₁₂-C₁₅
**Huile comparative 3** : Miglyol 812 : Triglycérides d'acides caprylique-caprique
**Huile comparative 4** : X-Tend : 2-phenyl ethyl benzoate

### Mode opératoire :

X mg de produit sont introduits dans Y mg d'huile ; avec un léger chauffage (< 60°C) et l'utilisation d'un sonicateur pendant 1 minute, la solution obtenue est laissée à température de labo pendant 1 mois ; l'état de cette solution est observée ; si aucun cristal ou dépôt huileux n'est visible, la solubilité du produit est considérée comme supérieure à Xx100/(X+Y) en poids/poids ; lorsque des cristaux ou un dépôt huileux apparaissent, l'essai est répété avec 5% en moins de produit.

**Tableau 1**

| **Solvant** | **Actif 1** | **Actif 2** | **Actif 3** |
|---|---|---|---|
| **Isopropyl N-lauroyl sarcosinate** | 23% | 18% | <2% Léger précipité |
| **Composé** **(m)** | 43% | 45% | 4% |

**Tableau 2**

| **Huile** | **Fil 1** | **Fil 2** | **Fil 3** | **Fil 4** | **Fil 5** | **Fil 6** | **Fil 7** | **Fil 8** |
|---|---|---|---|---|---|---|---|---|
| **Alkylbenzoate en C₁₂-C₁₅** | 15% | 25% | 30% | 49% | 28% | 60% | <4% Léger précipité | 15% |
| **Composé (m)** | 28% | 37% | 44% | 62% | 40% | 80% | 15% | 32% |

**Tableau 3**

| **Solvant** | **Fil 3** |
|---|---|
| **Isopropyl N-lauroyl sarcosinate** | 30% |
| **Alkylbenzoate en C₁₂-C₁₅** | 25% |
| **Triglycérides d'acides caprylique-caprique** | 22% |
| **2-phenyl ethyl benzoate** | 32% |
| **Composé (x) de l'invention** | 47% |
| **Composé (j) de l'invention** | 50% |
| **Composé (I) de l'invention** | 40% |
| **Composé (ii) de l'invention** | 39% |

### EXEMPLES DE FORMULATION 1-4

Les formulations suivantes ont été préparées, les quantités sont exprimées en pourcentages en poids par rapport au poids total de la composition.

| **Composition** | **Ex1** | **Ex2** | **Ex3** | **Ex4** |
|---|---|---|---|---|
| Phase A | | | | |
| Poly Dimethylsiloxane | 0,5 | 0,5 | 0,5 | 0,5 |
| ConservateursConservateurs | 1 | 1 | 1 | 1 |
| Acide Stéarique | 1,5 | 1,5 | 1,5 | 1,5 |
| Mélange mono-stéarate glycéryle / stéarate de PEG100 | 1 | 1 | 1 | 1 |
| Mélange de cétylstéaryl glucoside et d'alcools cétylique, stéarylique | 2 | 2 | 2 | 2 |
| Alcool cétylique | 0,5 | 0,5 | 0,5 | 0,5 |
| Butyl 1-butyl-5-oxopyrrolidine-3-carboxylate (composé (m)) | 20 | | 15 | - |
| Methyl 1-butyl-5-oxopyrrolidine-3-carboxylate (composé (j)) | | 20 | | 15 |
| 1-(4-methoxy-1-benzofuran-5-yl)-3-phenylpropane-1,3-dione | 5 | 5 | | |
| Dineopentyl 2-(3,3-diphenylprop-2-enylidene)malonate | - | - | 5 | 5 |

| Phase B | | | | |
|---|---|---|---|---|
| Eau désionisée | qsp 100 | qsp 100 | qsp 100 | qsp 100 |
| Glycérol | 5 | 5 | 5 | 5 |
| Gomme de xanthane | 0,2 | 0,2 | 0,2 | 0,2 |
| Phosphate de mono cétyle | 1 | 1 | 1 | 1 |

| Phase C | | | | |
|---|---|---|---|---|
| Isohexadecane | 1 | 1 | 1 | 1 |
| Copolymère acide acrylique/méthacrylate de stéaryle | 0,2 | 0,2 | 0,2 | 0,2 |
| Triethanolamine | qs pH | qs pH | qs pH | qsp H |

### Mode opératoire :

On chauffe la phase aqueuse (Phase B) contenant l'ensemble de ses ingrédients à 80°C au bain marie. On chauffe la phase grasse (Phase A) contenant l'ensemble de ses ingrédients à 80°C au bain marie. On émulsionne A dans B sous agitation de type rotor-stator (appareil de la société Moritz). On incorpore la Phase C et on laisse revenir à température ambiante sous agitation modérée. On introduit la triéthanolamine de façon à ajuster le pH à la valeur désirée en fin de fabrication. Les émulsions antisolaires obtenues sont stables au stockage et ne présentent pas de cristaux ou de précipités.

## Revendications

1. Utilisation d'au moins un dérivé ester de 2-pyrrolidinone 4-carboxy de formule (I) : 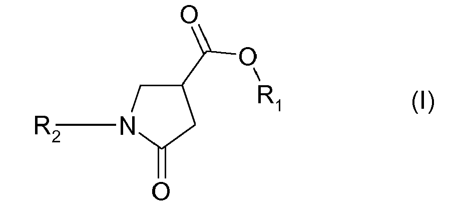 dans laquelle : .
R₁ désigne un radical alkyle en C₁-C₂₀, linéaire ou ramifié
R₂ désigne un radical alkyle en C₁-C₂₀, linéaire ou ramifié pouvant contenir un cycle en C₅-C₆, le radical phényle, le radical benzyle ou le radical phénéthyle, dans une composition comprenant dans un milieu cosmétiquement acceptable au moins une phase grasse liquide et au moins un actif lipophile, comme solvant dudit actif dans ladite phase grasse liquide et/ou comme agent améliorant la solubilité dudit actif dans ladite phase grasse.

2. Utilisation selon la revendication 1, où les composés de formule (I) sont choisis parmi les composés (a) à (oo) suivants :

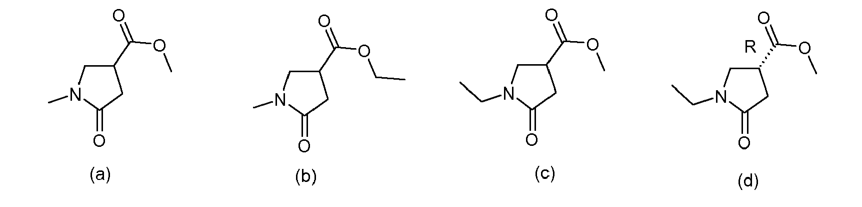

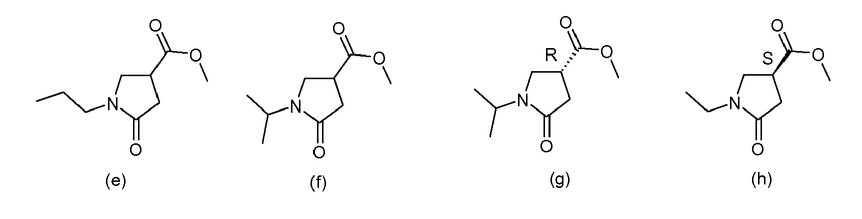

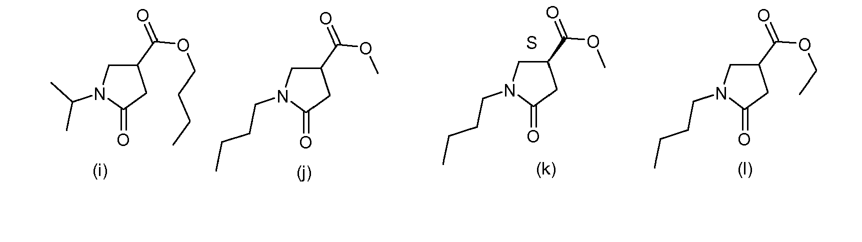

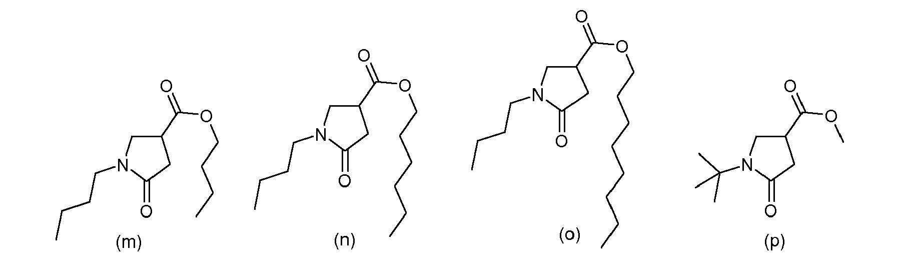

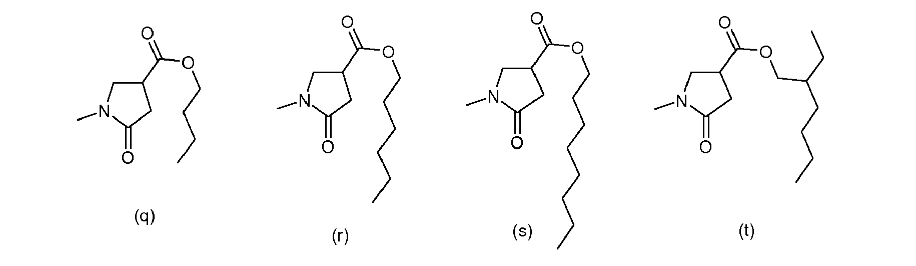

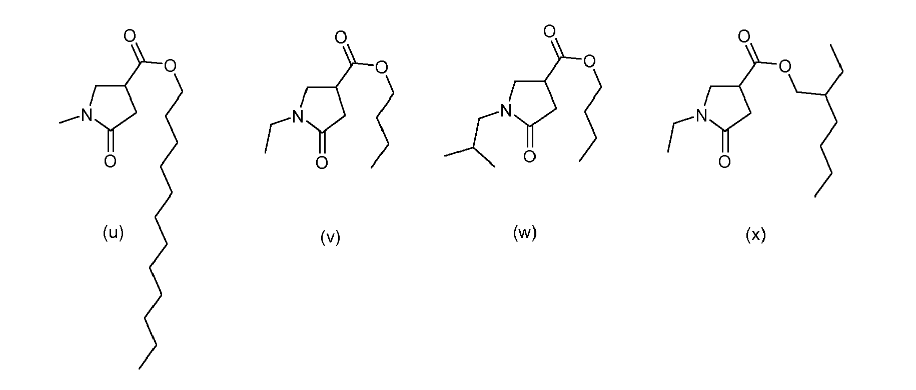

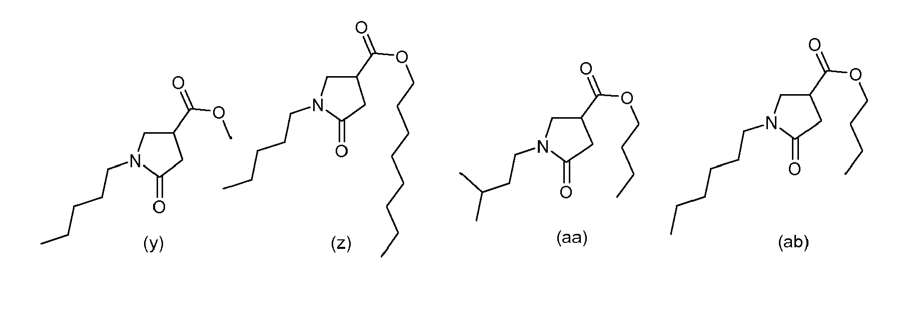

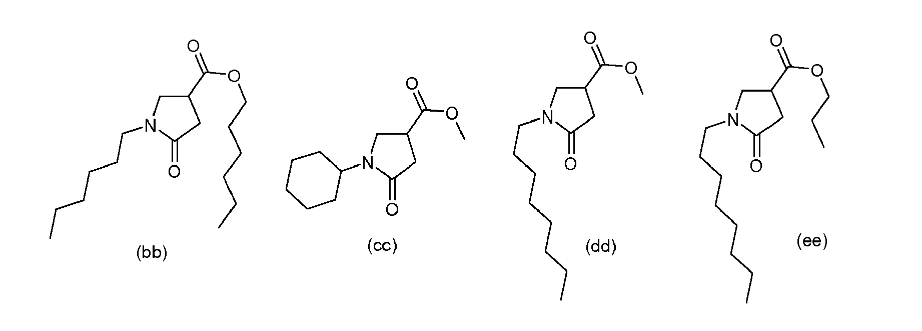

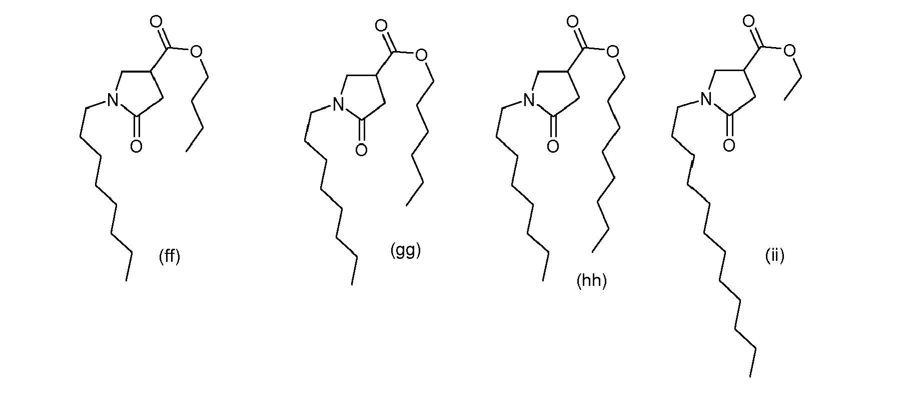

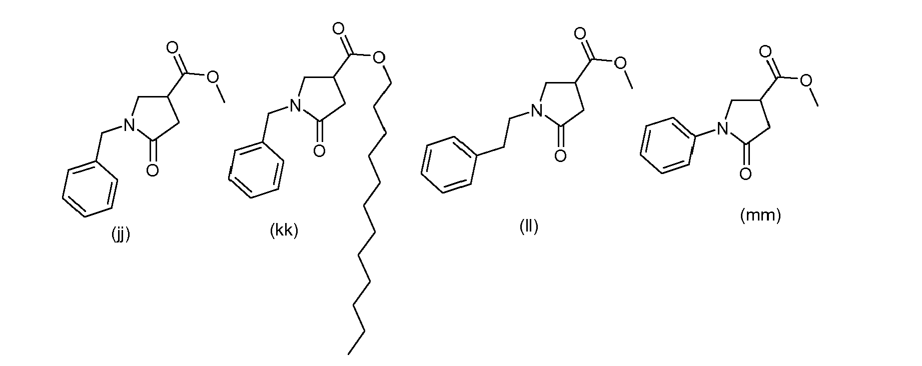

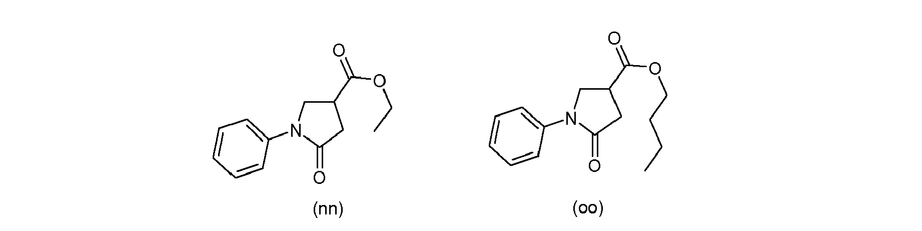

3. Utilisation selon la revendication 2, où les composés de formule (I) sont choisis parmi les composés (t), (u), (v), (x), (j), (l), (m), (w), (n), (ab), (ii) et (kk).

4. Utilisation selon l'une quelconque des revendications 1 à 3, où le ou les dérivés de formule (I) conformes à l'invention constitue(nt) l'unique solvant du ou des actif(s) lipophile(s).

5. Utilisation selon l'une quelconque des revendications 1 à 4, où l'actif lipophile est choisi parmi les filtres UV organiques lipophiles.

6. Utilisation selon la revendication 5, où le filtre lipophile est choisi parmi les dérivés de l'acide para-aminobenzoique, les dérivés salicyliques, les dérivés cinnamiques , les dérivés anthraniliques, les dérivés du dibenzoylméthane, les dérivés de β,β-diphénylacrylate, les dérivés du benzylidène camphre, les dérivés du phényl benzimidazole, les dérivés de benzotriazole, les dérivés d'imidazolines, les dérivés du benzalmalonate, les dérivés de 4,4-diarylbutadiène, les dérivés de benzoxazole, les chalcones, les dérivés de diphényl butadiène malonates ou malonitriles et leurs mélanges.

7. Utilisation selon la revendication 6, où les filtres UV organiques lipophiles sont choisis parmi les dérivés de dibenzoylméthane ; les benzotriazoles, les chalcones , les dérivés de diphényl butadiènes malonates ou malonitriles

8. Utilisation selon l'une quelconque des revendications 1 à 3, où l'actif lipophile est choisi parmi les dérivés d'aminophénol, les dérivés d'acide salicylique, les dérivés de N,N'-di(arylmethylene)ethylenediaminetriacetates, les dérivés 2-amino 4-alkylaminopyrimidine 3-oxyde, les flavonoïdes, le rétinol et ses dérivés, les carotenoïdes tel que le lycopène, et également les parfums, les huiles essentielles, les hormones, les vitamines en particulier la vitamine E, les céramides ou leurs mélanges.

9. Composition comprenant dans un milieu cosmétiquement acceptable au moins une phase grasse liquide **caractérisée par le fait qu'**elle contient au moins un dérivé ester de 2-pyrrolidinone 4-carboxy de formule (I) telle que défini dans l'une quelconque des revendications 1 et 2 et au moins un actif lipophile.

10. Composition selon la revendication 9, où l'actif lipophile est choisi parmi les filtres UV organiques lipophiles.

11. Composition selon la revendication 10, où le filtre lipophile est choisi parmi les dérivés de l'acide para-aminobenzoique, les dérivés salicyliques, les dérivés cinnamiques , les dérivés anthraniliques, les dérivés du dibenzoylméthane, les dérivés de β,β-diphénylacrylate, les dérivés du benzylidène camphre, les dérivés du phényl benzimidazole, les dérivés de benzotriazole, les dérivés d'imidazolines, les dérivés du benzalmalonate, les dérivés de 4,4-diarylbutadiène, les dérivés de benzoxazole, les chalcones, les dérivés de diphényl butadiène malonates ou malonitriles et leurs mélanges.

12. Composition selon la revendication 11, où le filtre UV organique lipophile est choisi parmi les benzotriazoles, les chalcones ; les dérivés de diphényl butadiènes malonates ou malonitriles.

13. Composition selon la revendication 8, où l'actif lipophile est choisi parmi les dérivés d'aminophénol, les dérivés d'acide salicylique, les dérivés de N,N'-di(arylmethylene)ethylenediaminetriacetates, les dérivés 2-amino 4-alkylaminopyrimidine 3-oxyde, les flavonoïdes, le rétinol et ses dérivés, les carotenoïdes tel que le lycopène, et également les parfums, les huiles essentielles, les hormones, les vitamines en particulier la vitamine E, les céramides ou leurs mélanges.

14. Composition selon l'une quelconque des revendications 8 à 13, où le ou les dérivés de formule (I) conformes à l'invention sont de préférence présents dans les compositions de l'invention à des teneurs allant de 1 à 30% en poids et plus préférentiellement de 3 à 20 % en poids par rapport au poids total de la composition.

15. Utilisation d'au moins un dérivé de formule (I) tel que défini dans l'une quelconque des revendications 1 et 2 dans une composition comprenant dans un milieu cosmétiquement acceptable au moins une phase grasse liquide et au moins un actif lipophile dans le but d'améliorer l'efficacité dudit actif et/ou les qualités cosmétiques et/ou la stabilité de ladite composition.

16. Utilisation d'au moins un dérivé de formule (I) tel que défini dans l'une quelconque des revendications 1 et 2 dans une composition comprenant dans un milieu cosmétiquement acceptable au moins une phase grasse liquide et au moins un filtre UV organique lipophile dans le but d'améliorer le facteur de protection solaire.
